# EUROPÄISCHE PATENTSCHRIFT

(11) **EP 4 064 990 B1**
(45) Veröffentlichungstag und Bekanntmachung des Hinweises auf die Patenterteilung: **10.04.2024**
(21) Anmeldenummer: 20816428.5
(22) Anmeldetag: 26.11.2020
(51) Int. Cl.: A61B 5/20

(54) **TOILETTENEINRICHTUNG ZUR BERÜHRUNGSFREIEN MESSUNG VON MIKTIONSPARAMETERN**
TOILET DEVICE FOR NON-CONTACT MEASUREMENT OF MICTURITION PARAMETERS
DISPOSITIF DE TOILETTES POUR LA MESURE SANS CONTACT DE PARAMÈTRES DE MICTION

(30) Priorität: 26.11.2019 DE 102019132005
(43) Veröffentlichungstag der Anmeldung: 05.10.2022
(73) Patentinhaber: MEDIPEE GMBH, 47445 Moers (DE)
(72) Erfinder: PROKOPP, Thomas, 53125 Bonn (DE); BANDI, Paul, 47475 Kamp-Lintfort (DE); MORCINCZYK, Marcel, 47495 Rheinberg (DE); HAAN, Jan, 47441 Moers (DE); WILLEMS, Frank, 47441 Moers (DE)
(74) Vertreter: FARAGO Patentanwälte GmbH
(86) Internationale Anmeldenummer: PCT/EP2020/083486
(87) Internationale Veröffentlichungsnummer: WO 2021/105272

(56) Entgegenhaltungen:
- WO-A1-2014/141234
- WO-A1-2017/036952
- US-A1- 2005 261 605

## Beschreibung

Die Erfindung betrifft eine Toiletteneinrichtung zur Messung von Miktionsparamatern, wobei die Toiletteneinrichtung ein Gehäuse mit einer Gehäuseöffnung zur Aufnahme von Urin und einen kapazitativen Sensor zur zeitabhängigen berührungslosen Messung von Miktionsparametern umfasst. Die Erfindung betrifft zudem ein Verfahren zur berührungslosen Messung von Miktionsparametern in einer Toiletteneinrichtung, das insbesondere unter Verwendung der erfindungsgemäßen Toiletteneinrichtung durchgeführt wird.

Die Ausscheidung von Urin, die auch als Urinieren, Wasserlassen, Blasenentleerung oder Miktion bezeichnet wird, ist physiologisch für den Körper aus mehreren Gründen sehr wichtig. Zum einen wird der Wasserhaushalt des Körpers auf diesem Wege reguliert. Zum anderen werden mit dem Urin Stoffe ausgeschieden, die beim Stoffwechsel anfallen und vom Körper nicht mehr benötigt werden. Dazu zählen auch giftige Substanzen, die über die Nahrung aufgenommen wurden, oder Medikamente. Durch die Untersuchung des Urins können Hinweise auf Krankheiten des Nieren- und Harnsystems entdeckt werden, aber auch Stoffwechselerkrankungen wie Diabetes oder Erkrankungen der Leber erkannt werden. Neben der eigentlichen Inhaltsanalyse des Urins kann auch eine Analyse von Harnflußparametern erfolgen. Hierzu wird die sogenannte Uroflowmetrie eingesetzt. Die Uroflowmetrie ist ein diagnostisches Verfahren, bei dem der Hamausfluss während der Blasenentleerung (Miktion) gemessen wird. Sie dient der objektiven Feststellung von Blasenentleerungsstörungen und gehört zu den Basisuntersuchungen in der Urologie. Durch die Messung des Harnstrahlvolumens pro Zeitspanne (z. B. in ml/s) wird eine Flußkurve erstellt, bei der die abgegebene Urinmenge über die Zeit aufgetragen wird. Diese Flusskurve zeigt bei bestimmten Erkrankungen einen typischen Verlauf. Nachteilig ist hier, dass diese Messung in der Klinik oder einer Praxis unter Anleitung durchgeführt werden muss und hierfür ein besonderes Untersuchungsgerät notwendig ist. Zudem ist es Voraussetzung für eine ordnungsgemäß durchgeführte Messung, dass die Blase des Probanden ausreichend gefüllt ist. Der Betroffene muss mit der Miktion (Blasenentleerung) warten, bis er ein starkes Druckgefühl verspürt. Das Ergebnis der Uroflowmetrie ist nur dann aussagekräftig, wenn die Urinmenge 150 ml übersteigt. Darüber hinaus kann das Sammeln des Urins über einen definierten Zeitraum (vorzugsweise 24h) notwendig sein, um vor allem bei Nierenkrankheiten die Funktionstüchtigkeit des Organs zu überprüfen. Häufig wird das abgegebene Urinvolumen im Rahmen von Transplantationen überprüft. Aufgabe der Erfindung ist es, ein verbessertes Verfahren und eine Toiletteneinrichtung zur Messung von Miktionsparametern zur Verfügung zu stellen.

Erfindungsgemäß wird diese Aufgabe durch eine Toiletteneinrichtung mit den Merkmalen des unabhängigen Anspruchs 1 gelöst. Vorteilhafte Weiterbildungen der Toiletteneinrichtung ergeben sich aus den Unteransprüchen 2 bis 9.

In einem weiteren Aspekt wird diese Aufgabe durch ein Verfahren zur Messung von Miktionsparametern mit den Merkmalen des Anspruchs 10 gelöst. Vorteilhafte Weiterbildungen des Verfahrens ergeben sich aus den Unteransprüchen 11 bis 13.

### Zusammenfassung der Erfindung

Die Erfindung stellt in einem ersten Aspekt eine Toiletteneinrichtung zur Messung von Miktionsparametern bereit, wobei diese Toiletteneinrichtung ein Gehäuse mit einer Gehäuseöffnung zur Aufnahme von Urin und einen kapazitativen Sensor zur zeitabhängigen Messung von Miktionsparametern umfasst, und wobei der kapazitative Sensor ein kapazitativer Näherungssensor ist.

Das erfindungsgemäße Verfahren vereinigt mehrere entscheidende Vorteile gegenüber den aus dem Stand der Technik bekannten Verfahren.

Durch die berührungslose Messung mittels des kapazitativen Näherungssensors wird der Sensor nicht durch Urin oder Kot kontaminiert. Es ist von daher keine zeit- und kostenaufwändige Reinigung des Sensors notwendig, sondern der Sensor ist ohne externe Eingriffe immer betriebsbereit.

Damit kann eine automatische digitalisierte Messung von Miktionsparametern erstmalig auch im häuslichen Umfeld durchgeführt werden. Der Proband oder Patient muss nicht erst in eine Praxis oder eine Klinik gehen, sondern die Messung kann in einfacher und für ihn ungestörter Weise in seinen Alltag integriert werden.

Durch die berührungslose Messung wiederum sinkt die Gefahr, dass Analyseergebnisse verfälscht werden. Zudem kann der kapazitative Sensor in einfacher Weise nachträglich an oder auf eine bestehende Toiletteneinrichtung angebracht werden und erlaubt so eine Nachrüstung einer bestehender Toiletteneinrichtung.

Weiterhin ist die erfindungsgemäße Messeinrichtung mit allen Gehäuse-tragenden Toiletteneinrichtungen kombinierbar und kann so in breiter Weise für unterschiedlichsten Toilettentypen eingesetzt werden.

Der kapazitative Sensor erlaubt in seiner einfachsten (d.h. nicht abgeschirmten) Variante eine raumunabhängige Messung und so kann neben der Messung der Miktionsparameter innerhalb des Gehäuses auch Vorgänge außerhalb der Toiletteneinrichtung, wie die Annäherung und das Hinsetzen des Toilettenbenutzers erfassen.

Die hier vorgeschlagenen Vereinfachungen der Vorrichtung bewirken nicht nur eine höhere Zuverlässigkeit der Vorrichtung, sondern reduzieren durch die hiermit einhergehende Bauteilreduzierung auch eine Gewichtsersparnis, sowie einen äußerst effizienteren Betrieb der Vorrichtung. Zudem erlauben sie eine unmittelbare Messung fast ohne Verzögerung.

Mit der Begrifflichkeit "Toiletteneinrichtung" ist im Sinne der Erfindung jegliche sanitäre Vorrichtung zur Aufnahme von Körperausscheidungen (insbesondere Kot und Urin) erfasst.

Unter dem Begriff "Gehäuse" wird erfindungsgemäß ein Gefäß als Bestandteil einer Toiletteneinrichtung verstanden, das den Urin aufnehmen kann. Das Gefäß weist bevorzugt eine wasserdichte (und damit urindichte) Gefäßwand auf, die besonders bevorzugt als steife und starre Wand ausgestaltet ist, so dass das Gehäuse beispielsweise als Sitz oder Sitzunterbau für den Toilettenbenutzer dienen kann. Das Gehäuse kann neben der für die Urinaufnahme vorgesehenen Gehäuseöffnung noch weitere Öffnungen aufweisen, wie beispielsweise für die Entsorgung der Exkremente oder die Zufuhr von Spülflüssigkeit.

Erfindungsgemäß ist der Begriff "Gehäuseöffnung" als diejenige Öffnung eines Gehäuses einer Toiletteneinrichtung definiert, durch die der Urin aufgenommen wird. Erfindungsgemäß ist damit nicht nur die Öffnung als solche erfasst, sondern auch ein Mittel, das zumindest auf einem Teil des Öffnungsrandes angebracht oder angeordnet ist und damit ein Teil der Öffnung bildet. Damit fällt auch eine Toilettenbrille als eine auf dem Rand aufsitzende Struktur unter den Begriff der Gehäuseöffnung. Erfindungsgemäß handelt es sich bei dem kapazitativen Sensor um einen kapazitiven Näherungssensor, der berührungsfrei - d. h. ohne direkten Kontakt - auf Annäherung von Flüssigkeiten mit einem elektrischen Sensor reagiert. Er nutzt die sich dabei ändernde elektrische Kapazität einer Messelektrode zur Umgebung oder einer Referenzelektrode aus. Die Einrichtung des kapazitativen Sensors ist erfindungsgemäß derart, dass die Kapazitätsänderung durch Einbringen eines elektrisch leitenden Materials, wie Urin oder eines Dielektrikums in die unmittelbare Umgebung des kapazitativen Sensors hervorgerufen wird. Gemäß der Erfindung ist unter der "unmittelbaren Umgebung" eine Umgebung in einem Abstand von weniger als 20 cm, bevorzugt weniger als 10 cm und weiterhin bevorzugt von weniger als 5 cm zu verstehen. Dies kann einerseits durch einen kapazitativen Sensor erreicht werden , bei dem zwei Elektroden die "Platten" eines elektrischen Kondensators bilden und wobei diese Elektroden/Platten in ihren Abstand und ihrer Orientierung fest zueinander angeordnet sind und dies auch dauerhaft der Fall ist. Durch die festgelegte Positionierung der Elektroden zueinander ändert sich die Kapazität, weil entweder elektrisch leitendes Material oder ein Dielektrikum in die unmittelbare Umgebung der Elektroden gebracht wird. Bei dem erfindungsgemäßen kapazitativen Sensor erfolgt somit keine Kapazitätsänderung durch Änderung der wirksamen Plattenfläche, indem z.B. die Platten wie bei einem Drehkondensator gegeneinander verschoben werden und auch keine Kapazitätsänderung dadurch, dass eine Platte durch den zu messenden Effekt verschoben oder verformt wird, wodurch sich der Plattenabstand und damit die elektrisch messbare Kapazität ändern. Die letztgenannten Messprinzipien erfordern im Gegensatz zu dem erfindungsgemäßen kapazitativen Sensor ein Inkontaktbringen des kapazitativen Sensors mit dem Messobjekt. Weiterhin kann der Sensor nur eine Elektrode aufweisen und es wird die Kapazität zwischen dieser aktiven Elektrode und dem elektrischen Erdpotential gemessen. Solche Sensoren arbeiten mit einem Oszillator, dessen frequenzbestimmende Kapazität teilweise vom zu detektierenden Medium bzw. der Umgebung gebildet wird. Bei Beeinflussung des Feldes der Sondenkapazität durch einen Leiter wie Urin beruht die Kapazitätsänderung auf einer Änderung der wirksamen Permittivität im Bereich der Elektroden; der erreichbare Schaltabstand beträgt üblicherweise 60-80 mm. Kapazitive Näherungssensoren haben in der Regel eine Kalibriermöglichkeit (Potentiometer), um die Empfindlichkeit bzw. die Schaltschwellen an die Einsatzbedingungen anzupassen. Ein weiteres Schaltungskonzept für einen erfindungsgemäßen kapazitativen Näherungssensor arbeitet mit drei Elektroden. Dabei wird neben der Masseelektrode und einer Messelektrode eine zusätzliche Anregungselektrode verwendet. Vorteile dieses Prinzips sind eine höhere Empfindlichkeit mit höheren Schaltabständen und eine geringere Störanfälligkeit. Es können damit auch Medien mit sehr geringer Permittivität oder größerem Abstand zum Sensor detektiert werden.

Gemäß der Erfindung ist der Begriff "kapazitativer Sensor" somit synonym mit dem Begriff "kapazitativer Näherungssensor".

Mit der erfindungsgemäßen Toiletteneinrichtung können alle physikalischen, d.h. alle direkt oder indirekt mit der Miktion zusammenhängenden Parameter gemessen werden. Bevorzugt sind die gemessenen Miktionsparameter hierbei ausgewählt aus der Gruppe aufweisend: Harnflussrate, Miktionsvolumen, Miktionsdauer, Miktionshäufigkeit, Miktionsgeschwindigkeit, Blasendruck, Beckenbodendruck, Harnröhrenweite und Miktionscharakteristika wie intermittierendes Urinieren oder Nachtröpfeln.

Da der kapazitative Sensor eine berührungslose Messung der Miktionsparameter erlaubt, kann er an allen möglichen Positionen in oder an der Toiletteneinrichtung angebracht werden. Bevorzugt ist der kapazitative Sensor in der Toiletteneinrichtung an einer oder mehrerer der folgenden Positionen angebracht:
- An der Gehäuse-Innenwand,
- An der Gehäuse-Außenwand,
- Im Inneren der Gehäusewand,
- An der Gehäuseöffnung,

Hierbei ist es besonders bevorzugt, dass der kapazitative Sensor an der Gehäuse-Außenwand angebracht ist. Ausgehend von einer üblichen Sitztoilette bedeutet diese eine Anbringung an der Außenseite der Toilettenschüssel.

In einer alternativen Ausführungsform ist der kapazitative Sensor an der Gehäuse-Innenwand angebracht. Diese Positionierung erlaubt durch ihre Nähe zum Urinstrahl eine besonders empfindliche Messung. In dieser Ausführungsform ist es vorteilhaft, wenn der kapazitative Sensor mit einer Schutzschicht abgedeckt ist, die wasserundurchlässig und urinresistent ist. Eine solche Schutzschicht weist zweckmäßigerweise eine hydrophobe Oberfläche auf, die bevorzugt eine Oberfläche mit einem Lotus-Effekt ist. Dadurch perlt der Urin von dem Sensor ab und der unbenetzte Sensor erlaubt weiterhin eine präzise Messung. Durch eine solche hydrophobe oder einen Lotus-Effekt aufweisende Oberfläche ist der kapazitative Sensor insbesondere in wasserfreien Toiletten und Urinalen geeignet.

In einer weiteren Ausführungsform ist der kapazitative Sensor an der Außenwand des Gehäuses angebracht. Diese Positionierung gewährleistet, dass der Sensor nicht mit den Exkrementen in Kontakt kommt. Die außenseitige Positionierung erlaubt auch ein einfaches Anbringen (z.B. im Rahmen eines Nachrüstens einer üblichen Toiletteneinrichtung), Warten, Reparieren, Entfernen oder Austauschen des Sensors.

In einer weiteren Ausführungsform ist der kapazitative Sensor im Inneren der Gehäusewand angebracht. Diese Positionierung zeichnet sich ebenfalls durch eine geringe Distanz zum Urinstrahl aus und ist daher auch für eine empfindliche Messung geeignet. Zudem ist der kapazitative Sensor damit durch den Einschluss oder das Einbringen in die Gehäusewand optimal vor Beschädigung oder Verschmutzung geschützt. Hierdurch bleibt die glatte (meist aus Keramik bestehende) Oberfläche der Toiletteneinrichtung unversehrt, so dass die Toiletteneinrichtung weiterhin einfach und gründlich gereinigt werden kann.

Erfindungsgemäß umfasst die Anbringung im Inneren der Gehäusewand auch eine Anbringung an einer Innenseite einer Gehäusewand, insofern diese Gehäusewand eine der beiden Wände einer doppelwandigen Gehäusewand darstellt. Bevorzugt ist der kapazitative Sensor hierbei an der Innenseite der zum Toiletteninneren zugewandten Gehäusewand angebracht. Im Sanitärkeramikbereich gibt es zahlreich Urinale oder Toiletten mit doppelwandigen Schüsseln.

Alternativ kann der Sensor bei der Herstellung des Gehäuses mit in die Wand eingebracht werden, so dass er vollständig von dem Wandmaterial umgeben ist. Dies kann beispielsweise bei einem Gehäuse aus Kunststoffmaterial durch fertigungsseitiges Eingießen in das Kunststoffgehäuse geschehen.

Zweckmäßigerweise weist der kapazitative Sensor eine Sensordicke von zwischen 1 nm und 20 mm auf. Kapazitative Sensoren können mit sehr geringer Dicke ausgeführt werden, so dass sie flexibel an die Rundungen des Gehäuses anpassbar sind und ohne Faltenbildung oder Knicke an das Gehäuse anbringbar sind. Die Sensordicke beträgt hierbei bevorzugt zwischen 1 nm und 20 mm, besonders bevorzugt zwischen 50 nm und 10 mm und insbesondere bevorzugt zwischen 200 nm und 3 mm.

In einer weiteren Ausführungsform umfasst der kapazitative Sensor eine untere Trägerschicht, eine mittlere die Elektrode und die Zuleitung(en) aufweisende Schicht und eine obere Montageschicht zur Verbindung des Sensors mit dem Gehäuse oder der Gehäuseöffnung, wobei die Trägerschicht und die Montageschicht bevorzugt aus einem elektrisch isolierenden Material bestehen.

Der kapazitative Sensor kann in eine isolierende Trägermatrix, wie Kunststoff oder einem festen Gel eingebracht sein. Hierbei ist bevorzugt, dass die Oberfläche der Trägermatrix zumindest teilweise eine Klebeschicht aufweist, die die Anbringung an das Gehäuse ermöglicht.

Der kapazitative Sensor umfasst zweckmäßigerweise ein Verbindungsmittel zur elektronischen und/oder elektrischen Verbindung des Sensors mit zusätzlichen elektronischen bzw. elektrischen Komponenten. Das Verbindungsmittel ist hierbei bevorzugt ausgewählt aus der Gruppe aufweisend metallischer Druckknopf, Kabel, Klebeverbindung und Steckerteil eines Steckverbinders.

Der kapazitative Sensor umfasst zweckmäßigerweise ein Datenübertragungsmodul zur kabelgebundenen oder kabellosen Datenübertragung. So können die erfassten Messdaten, also die Miktion betreffende Parameter, zu einer Speicher- und/oder Auswertungseinheit übermittelt werden.

In einer Ausführungsform ist der kapazitative Sensor als Folie ausgestaltet, was auch bei komplexen Gehäuseformen eine einfache und faltenfreie Anbringung erlaubt.

In einer weiteren Ausführungsform der Erfindung ist der Sensor direkt auf dem Gehäuse aufgedampft oder in das Gehäusematerial eingebrannt und stellt so einen integralen Bestandteil der Gehäusewand dar.

Erfindungsgemäß kann es auch vorgesehen sein, dass der kapazitative Sensor Elektroden aus einer dünnen Metallfolie umfasst, die auf einer elektrisch isolierenden Schicht aufgebracht sind. Eine solche isolierende Schicht besteht bevorzugt aus Gummi oder einem anderen Elastomer oder aus einem thermoplastischen Kunststoff. Besonders bevorzugt ist hierbei die Verwendung einer Polyimidfolie, die sowohl eine hohe Beständigkeit gegenüber Chemikalien, als auch eine hohe Temperaturbeständigkeit und gute Isoliereigenschaften aufweist. Im Handel sind zahlreiche Polyimidfolien bekannt, wie beispielsweise KAPTON der Firma DuPont.

Um auch kleine Veränderungen besser detektieren zu können, ist es vorteilhaft, wenn bei dem erfindungsgemäßen kapazitativen Sensor die eigentliche Messelektrode mit einer Schirmelektrode umgeben, die den inhomogenen Randbereich des elektrischen Felds von der Messelektrode abschirmt. Dadurch ergibt sich zwischen Messelektrode und üblicherweise geerdeter Gegenelektrode ein annähernd paralleles elektrisches Feld mit der bekannten Charakteristik eines idealen Plattenkondensators.

In einer weiteren Ausführungsform weist der kapazitative Sensor eine Abschirmung auf, die die störungsfreien Messung der Miktionsparameter in dem Gehäuse unterstützt.

In einer Ausführungsform kann es sich um eine als physische Barriere ausgeformte Abdeckung handeln, die an der Außenseite des Sensors angebracht ist, also an der vom Gehäuse abgewandten Seite der Toiletteneinrichtung.

Diese Abschirmung ist bevorzugt eine elektronische Abschirmung. Hierzu wird zweckmäßigerweise eine Abschirmelektrode verwendet.

In einer Ausführungsform ist die Abschirm-Elektrode parallel zur Messelektrode angebracht und wird durch eine separate Leitung angesteuert.

Die Abschirm-Elektrode dient als Schutz von externen Störsignalen, kann aber auch zur Verbesserung der Messgenauigkeit, zeitlichen Auflösung, Sensitivität, Selektivität und Messrichtung verwendet werden.

Das Steuersignal für die Abschirm-Elektrode kann gemäß einer Ausführungsform nur eine direkte Verbindung zur "Erde" (Grundpotential) sein, oder gemäß einer alternativen Ausführungsform aber auch angepasste Spannungssignale wie z.B.: das invertierte Steuersignal der Messelektrode bzw. alternierende Signale erhalten.

Andere Ansteuerungen der Abschirm-Elektrode sind ebenfalls denkbar und hängen vom Einsatzzweck ab. Gegebenenfalls wird noch eine zusätzliche Verstärkerschaltung benötigt. In einer weiteren Ausführungsform der Erfindung ist die Toiletteneinrichtung dadurch gekennzeichnet, dass mehrere kapazitative Sensoren in der Toiletteneinrichtung so angebracht sind, dass sie eine zweidimensionale oder dreidimensionale Messung der Miktion erlauben. Hierbei ist eine Anordnung in oder an dem Gehäuse als 2D-Array oder 3D-Array bevorzugt.

Gemäß der Erfindung kann der kapazitative Sensor fest, bedingt lösbar oder reversibel lösbar mit dem Gehäuse verbunden sein. Hierbei ist eine reversibel lösbare Verbindung bevorzugt, insofern der Sensor damit einfach ausgetauscht oder auf eine andere Toiletteneinrichtung übertragen werden kann. Für die Verbindung des Sensors mit dem Gehäuse stehen dem Fachmann zahlreiche Verbindungen zur Verfügung. Beispielhaft sind hier erwähnt: Klebeverbindung, klebstofffreie Adhäsionsverbindung, magnetische Verbindung, Klettverschluss, Reißverschluss, Schraubverbindung, Klemmverbindung, Steckverbindung, Schnappverbindung und Riemenverbindung. Bevorzugt ist hier die Klebeverbindung oder die klebstofffreie Adhäsionsverbindung.

In einer weiteren Ausführungsform der Erfindung ist die Toiletteneinrichtung dadurch gekennzeichnet, dass der kapazitative Sensor die Messung von außerhalb des Gehäuses der Toiletteneinrichtung vorliegenden Parametern erlaubt, wie beispielsweise Informationen zur Lokalisation des Benutzers der Toiletteneinrichtung und/oder die zeitliche und räumliche Veränderung der Benutzer-Lokalisation.

In einer bevorzugten Ausführungsform der Erfindung ist die Toiletteneinrichtung dadurch gekennzeichnet, dass sie eine Speicher- und/oder Auswertungseinheit (6) zur Speicherung bzw. Auswertung der Messdaten umfasst.

In einer Ausführungsform der Erfindung kann die Toiletteneinrichtung zusätzlich einen Temperatursensor und/oder einen Beschleunigungssensor umfassen. Bevorzugt ist hierbei, dass der Temperatursensor eine berührungslose Messung der Temperatur des Urins erlaubt. Bevorzugt ist weiterhin, dass der Beschleunigungssensor eine berührungslose Messung der benutzerseitigen Bewegungsdaten, Vibrationen und des Körperschalls während des Urinierens erlaubt.

Die Messung der Urintemperatur kann hierbei direkt oder indirekt erfolgen, also beispielsweise direkt über die Messung des Urinstrahls oder über die Messung des im Gehäuse ablaufenden Urinfilms, oder die Messung von Urinspritzern, Urinsprühnebel oder der Lufttemperatur in direkter Urinumgebung.

Bei der Messung der indirekten Urintemperatur ist es bevorzugt, eine zeitabhängige Auswertung der Messung vorzunehmen, da der Urin sich üblicherweise durch das Inkontaktbringen mit dem Gehäuse der Toiletteneinrichtung abkühlt und erst nach einigen Sekunden, nach entsprechender Erwärmung des Gehäuses eine aussagekräftige Urintemperatur erfasst werden kann.

Weiterhin ist es bevorzugt, dass bei der Messung der Urintemperatur weitere Temperaturdaten, wie beispielseiweise die Temperatur der Gehäusewand (oder Teilen hiervon) oder der Lufttemperatur erfasst werden und für die Auswertung der Urintemperatur herangezogen werden.

In diesem Zusammenhang ist es erfindungsgemäß auch vorgesehen, die Länge des Urinstrahls vom Körperaustritt bis zur Temperaturmessstelle zu erfassen, da der körperseitig austretende Urin sich durch die üblicherweise kältere Umgebungsluft abkühlt und die Abkühlung entsprechend mit der Länge des Urinstrahls zunimmt. Hierzu kann auch ein von der Toiletteneinrichtung angelegtes Benutzerprofil zum Urinierverhalten herangezogen werden.

In einer bevorzugten Ausführungsform ist der Temperatursensors für die berührungsfreie Messung als Infrarot-Sensor ausgestaltet. Er weist hierzu eine Infrarotquelle auf, die einen Bereich oder mehrere Bereiche innerhalb des Gehäuses der Toiletteneinrichtung anstrahlt und damit auf eine Zieltemperatur T_{ziel} erwärmt. Die durch die Miktion resultierende Änderung der Temperatur in diesem Bereich (diesen Bereichen) wird durch den Temperatursensor erfasst.

Der Temperatursensor ist bevorzugt an dem Gehäuse-Inneren oder der Gehäuseöffnung der Toiletteneinrichtung angebracht.

Da der Temperatursensor und/oder der Beschleunigungssensor in einer bevorzugten Ausgestaltung eine berührungslose Messung der Miktionsparameter erlaubt, kann er an allen möglichen Positionen in oder an der Toiletteneinrichtung angebracht werden. Bevorzugt ist der Temperatursensor und/oder der Beschleunigungssensor in der Toiletteneinrichtung an einer oder mehrerer der folgenden Positionen angebracht ist:
- An der Gehäuse-Innenwand,
- An der Gehäuse-Außenwand,
- Im Inneren der Gehäusewand,
- An der Gehäuseöffnung.

Hierbei ist es besonders bevorzugt, dass der Temperatursensor und/oder der Beschleunigungssensor an der Gehäuse-Außenwand angebracht ist. Ausgehend von einer üblichen Sitztoilette bedeutet diese eine Anbringung an der Außenseite der Toilettenschüssel.

In einer alternativen Ausführungsform ist der Temperatursensor und/oder der Beschleunigungssensor an der Gehäuse-Innenwand angebracht. Diese Positionierung erlaubt durch ihre Nähe zum Urinstrahl eine besonders empfindliche Messung. In dieser Ausführungsform sollte der Sensor mit einer Schutzschicht abgedeckt sein, die wasserundurchlässig und urinresistent ist. Eine solche Schutzschicht weist zweckmäßigerweise eine hydrophobe Oberfläche auf, die bevorzugt eine Oberfläche mit einem Lotus-Effekt ist. Dadurch perlt der Urin von dem Sensor ab und der unbenetzte Sensor erlaubt weiterhin eine präzise Messung. Durch eine solche hydrophobe oder einen Lotus-Effekt aufweisende Oberfläche ist der Temperatursensor und/oder der Beschleunigungssensor insbesondere in wasserfreien Toiletten und Urinalen geeignet.

In einer weiteren Ausführungsform ist der Temperatursensor und/oder der Beschleunigungssensor an der Außenwand des Gehäuses angebracht. Diese Positionierung gewährleistet, dass der Sensor nicht mit den Exkrementen in Kontakt kommt. Die außenseitige Positionierung erlaubt auch ein einfaches Anbringen (z.B. im Rahmen eines Nachrüstens einer üblichen Toiletteneinrichtung), Warten, Reparieren, Entfernen oder Austauschen des Sensors.

In einer weiteren Ausführungsform ist der Temperatursensor und/oder der Beschleunigungssensor im Inneren der Gehäusewand angebracht. Diese Positionierung zeichnet sich auch durch eine geringe Distanz zum Urinstrahl aus und ist daher auch für eine empfindliche Messung geeignet. Zudem ist der der Temperatursensor und/oder der Beschleunigungssensor damit durch den Einschluss oder das Einbringen in die Gehäusewand optimal vor Beschädigung oder Verschmutzung geschützt. Hierdurch wird die glatte (meist aus Keramik bestehende) Oberfläche der Toiletteneinrichtung aufrechterhalten, so dass die Toilette weiterhin einfach und gründlich gereinigt werden kann.

Erfindungsgemäß umfasst die Anbringung im Inneren der Gehäusewand auch eine Anbringung an einer Innenseite einer Gehäusewand, insofern diese Gehäusewand eine der beiden Wände einer doppelwandigen Gehäusewand darstellt. Bevorzugt ist der Temperatursensor und/oder der Beschleunigungssensor hierbei an der Innenseite der zum Toiletteninneren zugewandten Gehäusewand angebracht. Im Sanitärkeramikbereich gibt es zahlreich Urinale oder Toiletten mit doppelwandigen Becken.

Alternativ kann der der Temperatursensor und/oder der Beschleunigungssensor bei der Herstellung des Gehäuses mit in die Wand eingebracht werden, so dass er vollständig von dem Wandmaterial umgeben ist. Dies kann beispielsweise bei einem Gehäuse aus Kunststoffmaterial durch fertigungsseitiges Eingießen in das Kunststoffgehäuse geschehen.

Zweckmäßigerweise weist der Temperatursensor und/oder der Beschleunigungssensor eine Sensordicke von zwischen 1 nm und 20 mm auf. Kapazitative Sensoren können mit sehr geringer Dicke ausgeführt werden, so dass sie flexibel an die Rundungen des Gehäuses anpassbar und ohne Faltenbildung oder Knicke an das Gehäuse anbringbar sind. Die Sensordicke beträgt hierbei bevorzugt zwischen 50 nm und 10 mm und besonders bevorzugt zwischen 200 nm und 3 mm.

In einer weiteren Ausführungsform umfasst der Temperatursensor und/oder der Beschleunigungssensor eine untere Trägerschicht, eine mittlere die Messelektronik und die Zuleitung(en) aufweisende Schicht und eine obere Montageschicht zur Verbindung des Temperatursensors und/oder des Beschleunigungssensors mit dem Gehäuse oder der Gehäuseöffnung, wobei die Trägerschicht und die Montageschicht bevorzugt aus einem elektrisch isolierenden Material bestehen.

Der Temperatursensor und/oder der Beschleunigungssensor kann in eine isolierende Trägermatrix, wie Kunststoff oder einem festen Gel eingebracht sei. Hierbei ist bevorzugt, dass die Oberfläche der Trägermatrix zumindest teilweise eine Klebeschicht aufweist, die die Anbringung an das Gehäuse ermöglicht.

Der Temperatursensor und/oder der Beschleunigungssensor umfasst zweckmäßigerweise ein Verbindungsmittel zur elektronischen oder elektrischen Verbindung des Sensors mit zusätzlichen elektronischen oder elektrischen Komponenten. Das Verbindungsmittel ist hierbei bevorzugt ausgewählt aus der Gruppe aufweisend metallischer Druckknopf, Klebeverbindung, Kabel und Steckerteil eines Steckverbinders.

Der Temperatursensor und/oder der Beschleunigungssensor umfasst/umfassen zweckmäßigerweise ein Datenübertragungsmodul zur kabelgebundenen oder kabellosen Datenübertragung. So können die erfassten Messdaten als die Miktion betreffenden Parameter, zu einer Speicher- und/oder Auswertungseinheit übermittelt werden.

Weitere Daten der hierin beanspruchten Sensoren können kumulativ oder alternativ an eine Analyseeinheit gesendet werden, welche beispielsweise in dem Gehäuse der Vorrichtung und/oder aber auch an dem Führungsteil der Vorrichtung sitzen kann.

Ferner ist es möglich, dass kumulativ oder alternativ Daten von den Sensoren und/oder von der Auswertungseinheit an eine externe Analyseeinheit übermittelt werden. Gedacht sei hierbei nur zum Beispiel an eine auf einem Smartphone oder dergleichen laufende Datenverarbeitung- und/oder Analyseapplikation.

Es ist hierbei denkbar, dass zumindest einige der Sensoren je nach zu analysierendem Urin ab- oder zugeschaltet werden können, wodurch die Vorrichtung energetisch effizienter betrieben werden kann.

Es versteht sich, dass vor Ort gewonnene Daten bzw. Informationen zur weiteren Auswertung oder zur Speicherung aber auch an externe Einrichtungen, wie etwa einem Datenträger usw., übermittelt werden können.

In einer Ausführungsform ist der Temperatursensor und/oder der Beschleunigungssensor als Folie ausgestaltet, was auch bei komplexen Gehäuseformen eine einfache und oberflächenbündige, d.h. faltenfreie und blasenfreie Anbringung erlaubt.

In einer weiteren Ausführungsform der Erfindung ist der Temperatursensor und/oder der Beschleunigungssensor direkt auf dem Gehäuse aufgedampft oder in das Gehäusematerial eingebrannt und stellt so einen integralen Bestandteil der Gehäusewand dar.

In einer weiteren Ausführungsform der Erfindung ist die Toiletteneinrichtung dadurch gekennzeichnet, dass mehrere Temperatursensoren bzw. Beschleunigungssensoren in der Toiletteneinrichtung so angebracht sind, dass sie eine zweidimensionale oder dreidimensionale Messung der Temperatur bzw. Beschleunigung erlauben. Hierbei ist eine Anordnung in oder an dem Gehäuse als 2D-Array oder 3D-Array bevorzugt.

Gemäß der Erfindung kann der der Temperatursensor und/oder der Beschleunigungssensor fest, bedingt lösbar oder reversibel lösbar mit dem Gehäuse verbunden sein. Hierbei ist eine reversibel lösbare Verbindung bevorzugt, insofern der Temperatursensor und/oder der Beschleunigungssensor damit einfach ausgetauscht oder auf eine andere Toiletteneinrichtung übertragen werden kann. Für die Verbindung des Sensors mit dem Gehäuse stehen dem Fachmann zahlreiche Verbindungen zur Verfügung. Beispielhaft sind hier erwähnt: Klebeverbindung, klebstofffreie Adhäsionsverbindung, magnetische Verbindung, Klettverschluss, Reißverschluss, Schraubverbindung, Klemmverbindung, Steckverbindung, Schnappverbindung und Riemenverbindung. Bevorzugt ist hier die Klebeverbindung oder die klebstofffreie Adhäsionsverbindung.

In einer weiteren Ausführungsform der Erfindung ist die Toiletteneinrichtung dadurch gekennzeichnet, dass der Temperatursensor, der bevorzugt zur berührungslosen Messung der Urintemperatur eingerichtet ist, weiterhin bevorzugt einer oder mehrere der folgenden Eigenschaften aufweist:
- Der Temperatursensor ist so eingerichtet, dass er die Messung der Strahltemperatur während des Urinierens erlaubt;
- Der Temperatursensor ist so eingerichtet, dass er die Messung der Oberflächentemperatur der Innenseite des Gehäuses und ihrer zeitlichen Veränderung erlaubt;
- Der Temperatursensor ist ein Infrarotsensor;
- Der Temperatursensor ist ein 1D-, 2D- oder 3D-Sensor.

In einem zweiten Aspekt stellt die Erfindung ein Verfahren zur Messung von Miktionsparametern in einer Toiletteneinrichtung bereit, wobei die Toiletteneinrichtung ein eine Gehäuseöffnung aufweisendes Gehäuse und einen kapazitativen Näherungssensor umfasst, wobei das Verfahren die folgenden Schritte umfasst:
a) Berührungslose Messung der Miktionsparameter während des Urinierens durch den kapazitativen Näherungssensor;
b) Optionale Messung der Urintemperatur während des Urinierens über einen zusätzlich in der Toiletteneinrichtung eingebrachten Temperatursensor;
c) Optionale Messung der benutzerseitigen Bewegungsdaten, Vibrationen und des Körperschalls während des Urinierens über einen zusätzlich in der Toiletteneinrichtung eingebrachten Beschleunigungssensor;
d) Kabelgebundene oder kabellose Übertragung der in den Schritten a) bis c) erhobenen Messdaten an eine Auswertungseinheit; und
e) Auswertung der Messdaten in der Auswertungseinheit.

In einer bevorzugten Ausführungsform der Erfindung wird das vorab beschriebene Verfahren mit der erfindungsgemäßen Toiletteneinrichtung durchgeführt.

In einer weiteren Ausführungsform erfolgt bei dem erfindungsgemäßen Verfahren die Auswertung der Messdaten in der Auswertungseinheit modellbasiert oder über Kalibrationskurven. Die modellbasierte Auswertung beruht hierbei bevorzugt auf einem mathematischen, physikalischen, physiologischen oder medizinischen Modell oder sie verläuft über Mustererkennungsalgorithmen.

In einer Ausführungsform der Erfindung weist die Toilettenanlage zusätzlich eine WC-Spülung auf, deren Charakteristika wie Spülwassermenge und/oder Spüldauer und/oder Spülwassertemperatur als Referenzwerte für die Auswertung herangezogen werden.

In einem dritten Aspekt betrifft die Erfindung die Verwendung eines kapazitativen Sensors zur Messung von Miktionsparametern in einer Toiletteneinrichtung. Bevorzugt wird hierbei der Sensor zur Messung der Miktionsparameter in der erfindungsgemäßen Toiletteneinrichtung verwendet. Ebenfalls bevorzugt erfolgt bei dieser Verwendung die Messung der Miktionsparameter nach dem vorab beschriebenen erfindungsgemäßen Verfahren.

An dieser Stelle sei noch erwähnt, dass im Rahmen der hier vorliegenden Patentanmeldung der Ausdruck "insbesondere" immer so zu verstehen sei, dass mit diesem Ausdruck ein optionales, bevorzugtes Merkmal eingeleitet wird. Der Ausdruck ist nicht als "und zwar" und nicht als "nämlich" zu verstehen.

Ferner sei darauf hingewiesen, dass im Rahmen der hier vorliegenden Patentanmeldung unbestimmte Artikel und unbestimmte Zahlenangaben wie "ein...", "zwei..." usw. im Regelfall als mindestens-Angaben zu verstehen sein sollen, also als "mindestens ein...", "mindestens zwei..." usw., sofern sich nicht etwa aus dem Kontext oder dem konkreten Text einer bestimmten Stelle ergibt, dass etwa dort nur "genau ein...", "genau zwei..." usw. gemeint sein soll.

Zusätzlich sind weitere Merkmale, Effekte und Vorteile vorliegender Erfindung anhand anliegender Zeichnung und nachfolgender Beschreibung erläutert, in welchen beispielhaft eine Vorrichtung zur Vor-Ort-Analyse von Exkrementen dargestellt und beschrieben ist.

Komponenten, welche in den einzelnen Figuren wenigstens im Wesentlichen hinsichtlich ihrer Funktion übereinstimmen, können hierbei mit gleichen Bezugszeichen gekennzeichnet sein, wobei die Komponenten nicht in allen Figuren beziffert und erläutert sein müssen.

Es sei hier gesagt, dass es sich bei den gezeigten Figuren um Darstellungen handelt, welche den prinzipiellen Aufbau und die prinzipielle Funktionsweise illustrieren.

In der Zeichnung zeigen:
- Figur 1: schematisch eine erste perspektivische Ansicht einer teilweise dargestellten Toiletteneinrichtung gemäß einer Ausführungsform der Erfindung mit einem 2D-Array an kapazitativen Sensoren;
- Figur 2: schematisch eine perspektivische Ansicht einer teilweise dargestellten Toiletteneinrichtung gemäß einer zweiten Ausführungsform der Erfindung mit einer Abdeckung der außenseitig angebrachten kapazitativen Sensoren;
- Figur 3: schematisch eine perspektivische Ansicht einer teilweise dargestellten Toiletteneinrichtung gemäß einer weiteren Ausführungsform der Erfindung mit einem Temperatursensor.

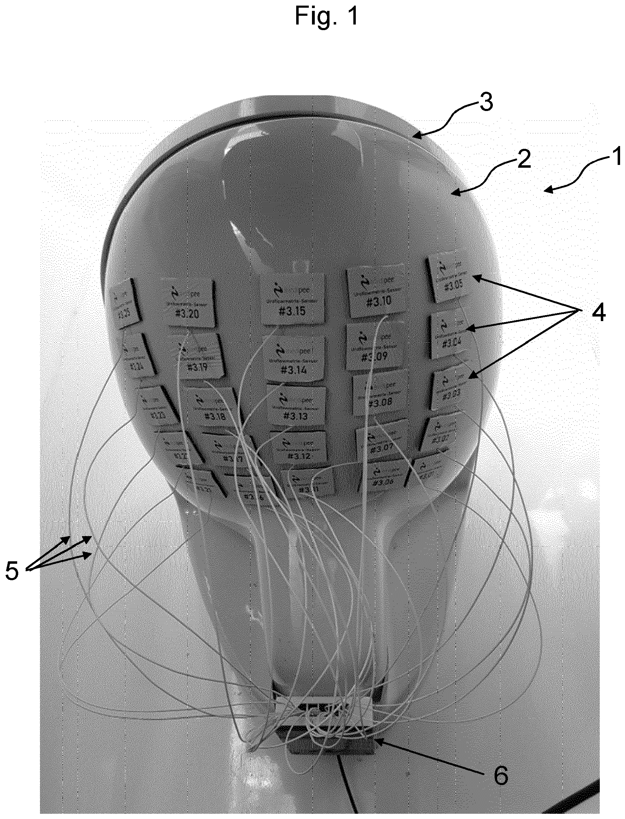

Figur 1 zeigt eine Toiletteneinrichtung 1 mit einer Toilettenschüssel 2 und einer Toilettenbrille 3. Die kapazitativen Sensoren 4 sind als 2D-Array aus 5 x 5 Sensoren in nahezu quadratischer Anordnung auf der Außenwand der Toilettenschüssel angeklebt und jeweils durch ein Kabel 5 mit der Stromquelle mit Auswertungs- und Datenübertragungseinheit 6 verbunden.

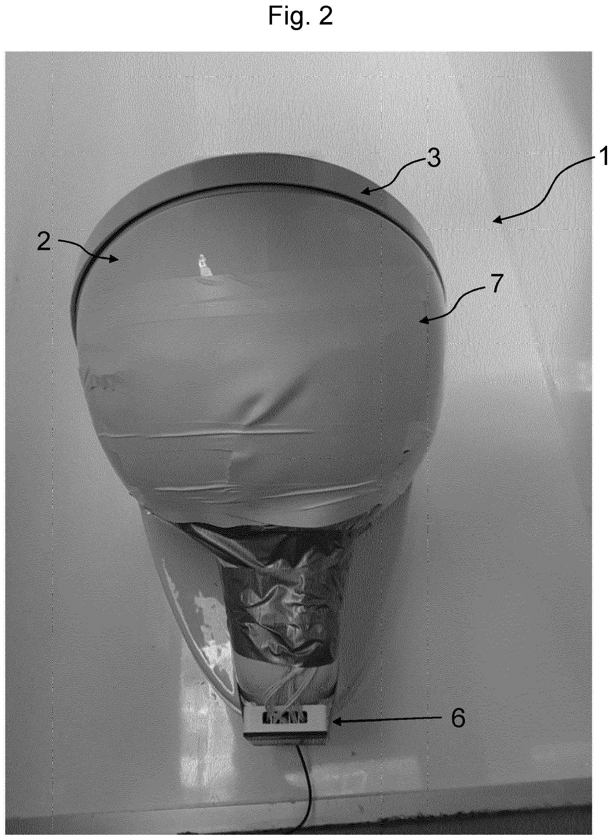

Figur 2 zeigt die Toiletteneinrichtung 1 gemäß Figur 1 mit einer Toilettenschüssel 2 und einer Toilettenbrille 3. Die Stromquelle mit Auswertungs- und Datenübertragungseinheit 6 verbundenen kapazitativen Sensoren 4 sind hier durch eine Abdeckung 7 vollständig und wasserdicht abgedeckt.

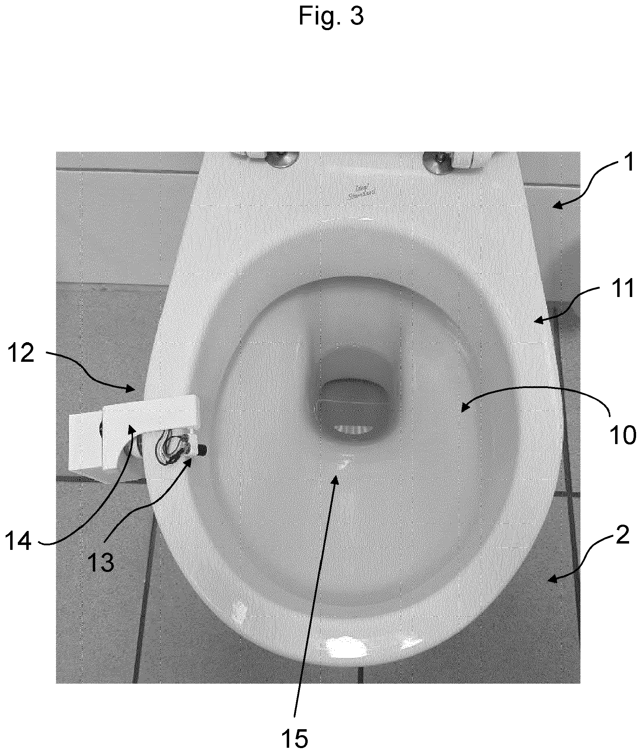

Figur 3 zeigt eine Toiletteneinrichtung 1 mit einer Toilettenschüssel 2 und deren Öffnung 10. Durch das Hochklappen der auf dem Schüsselrand 11 aufliegenden Toilettenbrille 3 (hier nicht dargestellt), wir der Temperatursensor 12 vollständig sichtbar, der mittels der als Klemmvorrichtung ausgebildeten Haltevorrichtung 14 an der Wand der Toilettenschüssel befestigt ist. Der Temperatursensor 12 weist einen Kopf 13 auf, der einen Infrarotsensor beinhaltet, der auf den Messpunkt 15 ausgerichtet ist und dort die Messung der Temperatur erlaubt.

Es versteht sich, dass es sich bei dem vorstehend erläuterten Ausführungsbeispiel lediglich um eine erste Ausgestaltung der erfindungsgemäßen Vorrichtung handelt. Insofern beschränkt sich die Ausgestaltung der Erfindung durch die Gegenstand der Ansprüche.

Liste der verwendeten Bezugszeichen
- 1: Toiletteneinrichtung
- 2: Gehäuse (Toilettenschüssel)
- 3: Toilettenbrille
- 4: kapazitative Näherungssensoren
- 5: Kabel
- 6: Stromquelle mit Auswertungs- und Datenübertragungseinheit
- 7: Sensorabdeckung
- 10: Gehäuseöffnung
- 11: Gehäuserand (Rand der Toilettenschüssel)
- 12: Temperatursensor
- 13: Kopf des Temperatursensors
- 14: Haltevorrichtung für Temperatursensor
- 15: Temperatur-Meßpunkt

## Patentansprüche

1. Toiletteneinrichtung (1) zur Messung von Miktionsparametern, umfassend:
• Ein Gehäuse (2) mit einer Gehäuseöffnung (10) zur Aufnahme von Urin;
• Einen kapazitiven Sensor (4) zur zeitabhängigen Messung von Miktionsparametern,
wobei der kapazitive Sensor (4) ein kapazitiver Näherungssensor ist, der berührungsfrei auf die Annäherung von Flüssigkeiten reagiert,
wobei die Toiletteneinrichtung (1) ausgewählt ist aus der Gruppe aufweisend Toilette, Urinal, Bidet, Toilettenstuhl, Hocktoilette und Nachttopf.

2. Toiletteneinrichtung (1) gemäß Anspruch 1, **dadurch gekennzeichnet, dass** die gemessenen Miktionsparameter ausgewählt sind aus der Gruppe aufweisend Harnflussrate, Miktionsvolumen, Miktionsdauer, Miktionshäufigkeit, Miktionsgeschwindigkeit, Blasendruck, Beckenbodendruck, Harnröhrenweite und Miktionscharakteristika wie intermittierendes Urinieren oder Nachtröpfeln.

3. Toiletteneinrichtung (1) gemäß einem der Ansprüche 1 bis 2, **dadurch gekennzeichnet, dass** der kapazitive Sensor (4) in der Toiletteneinrichtung (1) an einer oder mehrerer der folgenden Positionen angebracht ist:
• An der Gehäuse-Innenwand,
• An der Gehäuse-Außenwand,
• Im Inneren der Gehäusewand,
• An der Gehäuseöffnung (10),
wobei die Anbringung an der Gehäuse-Außenwand bevorzugt ist.

4. Toiletteneinrichtung (1) gemäß einem der vorangehenden Ansprüche, **dadurch gekennzeichnet, dass** der kapazitive Sensor (4) eine oder mehrere der folgenden Eigenschaften aufweist:
• Sensordicke zwischen 1 nm und 20 mm,
• der Sensor umfasst eine untere Trägerschicht, eine mittlere die Elektrode und die Zuleitung aufweisende Schicht und eine obere Montageschicht zur Verbindung des Sensors mit dem Gehäuse oder der Gehäuseöffnung, wobei die Trägerschicht und die Montageschicht bevorzugt aus einem elektrisch isolierenden Material bestehen;
• der Sensor ist in eine isolierende Trägermatrix, wie Kunststoff oder einem festen Gel eingebracht, wobei die Oberfläche der Trägermatrix zumindest teilweise eine Klebeschicht aufweist;
• Der Sensor umfasst ein Verbindungsmittel zur elektronischen oder elektrischen Verbindung, das bevorzugt ausgewählt ist aus der Gruppe aufweisend metallischer Druckknopf, Klebeverbindung, Kabel und Steckerteil eines Steckverbinders;
• Der Sensor umfasst ein Datenübertragungsmodul zur kabelgebundenen oder kabellosen Datenübertragung;
• Der Sensor ist als Folie ausgestaltet;
• Der Sensor ist direkt auf dem Gehäuse aufgedampft oder in das Gehäusematerial eingebrannt;
• Der Sensor umfasst Elektroden aus einer dünnen Metallfolie, die auf einer elektrisch isolierenden Schicht, die beispielsweise aus Gummi oder einem flexiblen Kunststoff wie Polyimidfolie besteht, aufgebracht sind.

5. Toiletteneinrichtung (1) gemäß einem der vorangehenden Ansprüche, **dadurch gekennzeichnet, dass** mehrere kapazitive Sensoren (4) in der Toiletteneinrichtung (1) so angebracht sind, dass sie eine zweidimensionale oder dreidimensionale Messung der Miktion erlauben, und hierbei bevorzugt in oder an dem Gehäuse als 2D-Array oder 3D-Array angeordnet sind.

6. Toiletteneinrichtung (1) gemäß einem der vorangehenden Ansprüche, **dadurch gekennzeichnet, dass** der kapazitive Sensor (4) fest, bedingt lösbar oder reversibei lösbar mit dem Gehäuse(2) verbunden ist, wobei eine reversibel lösbare Verbindung bevorzugt ist und reversibel lösbare Verbindung besonders bevorzugt ausgewählt ist aus der Gruppe aufweisend: Saugnapf, Klebeverbindung, klebstofffreie Adhäsionsverbindung, magnetische Verbindung, Klettverschluss, Reißverschluss, Schraubverbindung, Klemmverbindung, Steckverbindung, Schnappverbindung und Riemenverbindung

7. Toiletteneinrichtung (1) gemäß einem der vorangehenden Ansprüche, **dadurch gekennzeichnet, dass** der kapazitive Sensor (4) die Messung von außerhalb des Gehäuses (2) vorliegenden Parametern erlaubt, wie beispielsweise Informationen zur Lokalisation des Benutzers der Toiletteneinrichtung und ihre zeitliche und räumliche Veränderung.

8. Toiletteneinrichtung (1) gemäß einem der vorangehenden Ansprüche, **dadurch gekennzeichnet, dass** sie zusätzlich einen Temperatursensor (12) und/oder einen Beschleunigungssensor umfasst.

9. Toiletteneinrichtung (1) gemäß einem der vorangehenden Ansprüche, **dadurch gekennzeichnet, dass** der Temperatursensor (12) zur berührungslosen Messung der Urintemperatur eingerichtet ist, und bevorzugt einer oder mehrere der folgenden Eigenschaften aufweist:
• Der Temperatursensor (12) ist so eingerichtet, dass er die Messung der Urintemperatur während des Urinierens erlaubt;
• Der Temperatursensor (12) ist so eingerichtet, dass er die Messung der Oberflächentemperatur der Innenseite des Gehäuses und ihrer zeitlichen Veränderung erlaubt;
• Der Temperatursensor (12) ist ein Infrarotsensor;
• Der Temperatursensor (12) ist ein 1D-, 2D- oder 3D-Sensor.

10. Verfahren zur Messung von Miktionsparametern in einer Toiletteneinrichtung (1) aufweisend ein eine Gehäuseöffnung (10) aufweisendes Gehäuse (2) und einen kapazitiven Näherungssensor (4), wobei das Verfahren die folgenden Schritte umfasst:
a) Berührungslose Messung der Miktionsparameter während des Urinierens durch den kapazitiven Näherungssensor (4);
b) Optionale Messung der Urintemperatur während des Urinierens über einen für diese Option optional zusätzlich in der Toiletteneinrichtung eingebrachten Temperatursensor (12);
c) Optionale Messung der benutzerseitigen Bewegungsdaten, Vibrationen und des Körperschalls während des Urinierens über einen für diese Option zusätzlich in der Toiletteneinrichtung eingebrachten Beschleunigungssensor;
d) Kabelgebundene oder kabellose Übertragung von in den Schritten a) bis c) erhobenen Messdaten an eine Auswertungseinheit (6);
e) Auswertung der Messdaten in der Auswertungseinheit.

11. Verfahren gemäß Anspruch 10, **dadurch gekennzeichnet, dass** das Verfahren mit einer Toiletteneinrichtung (1) gemäß einem der Ansprüche 1 bis 10 durchgeführt wird.

12. Verfahren gemäß Anspruch 10 oder 11, **dadurch gekennzeichnet, dass** die Auswertung der Messdaten in der Auswertungseinheit (6) modellbasiert oder über Kalibrationskurven erfolgt, wobei die modellbasierte Auswertung bevorzugt auf einem mathematischen, physikalischen, physiologischen oder medizinischen Modell beruht oder über Mustererkennungsalgorithmen verläuft.

13. Verfahren gemäß Anspruch 10 bis 12, **dadurch gekennzeichnet, dass** die Toiletteneinrichtung (1) zusätzlich eine WC-Spülung aufweist, deren Charakteristika wie Spülwassermenge und/oder Spüldauer und/oder Spülwassertemperatur als Referenzwerte für die Auswertung herangezogen werden.

14. Verwendung eines kapazitiven Näherungssensors (4) zur Messung von Miktionsparametern in einer Toiletteneinrichtung (1),
wobei der kapazitive Näherungssensor berührungsfrei auf die Annäherung von Flüssigkeiten reagiert,
wobei die Toiletteneinrichtung (1) ausgewählt ist aus der Gruppe aufweisend Toilette, Urinal, Bidet, Toilettenstuhl, Hocktoilette und Nachttopf.

## Claims

1. Toilet device (1) for measuring micturition parameters, comprising:
• A housing (2) with a housing opening (10) for taking in urine;
• A capacitative sensor (4) for time-dependent measurement of micturition parameters,
whereby the capacitative sensor (4) is a capacitative proximity sensor that reacts without contact to the approach of fluids.
whereby the toilet device (1) is selected from the group comprising toilet, urinal, bidet, commode, squat toilet and chamber pot.

2. A toilet device (1) according to Claim 1, **characterized in that** the measured micturition parameters are selected from the group comprising urine flow rate, micturition volume, micturition duration, micturition frequency, micturition speed, bladder pressure, pelvic floor pressure, urethral width and micturition characteristics such as intermittent urination or dribbling.

3. A toilet device (1) according to Claims 1 to 2, **characterized in that** the capacitative sensor (4) in the toilet device (1) is attached in one or more of the following positions:
• On the internal wall of the housing,
• On the external wall of the housing,
• Inside the housing wall,
• On the housing opening (10),
whereby attachment to the external wall of the housing is preferential.

4. A toilet device (1) according one of the preceding claims, **characterized in that** the capacitative sensor (4) has one or more of the following characteristics:
• Sensor thickness between 1 nm and 20 mm,
• the sensor comprises a lower support layer, a middle layer containing the electrode and the lead and an upper mounting layer for connecting the sensor to the housing or the housing opening, the support layer and the mounting layer preferentially consist of an electrically insulating material;
• the sensor is incorporated into an insulating support matrix, such as plastic or a solid gel, the surface of the support matrix at least partially having an adhesive layer;
• the sensor comprises a connecting means for electronic or electrical connection, which is preferentially selected from the group comprising a metallic push button, adhesive connection, cable and male part of a plug connector;
• the sensor comprises a data transfer module to wired or wireless data transfer;
• the sensor is designed as a film;
• the sensor is vapor-deposited directly onto the housing or burned into the housing material;
• the sensor comprises electrodes made of a thin metal foil, which are applied to an electrically insulating layer, consisting, for example, of rubber or a flexible plastic such as polyimide film.

5. A toilet device (1) according to one of the preceding claims, **characterized in that** a plurality of capacitive sensors (4) are mounted in the toilet device (1) in such a way that they allow two-dimensional or three-dimensional measurement of micturition, and hereby preferentially arranged in or on the housing as a 2D array or 3D array.

6. A toilet device (1) according to one of the preceding claims, **characterized in that** the capacitive sensor (4) is connected to the housing (2) in a fixed, conditionally detachable or reversibly detachable manner, a reversibly detachable connection being preferred and a reversibly detachable connection being particularly preferably selected from the group comprising: suction cup, adhesive connection, adhesive-free adhesion connection, magnetic connection, hook-and-loop fastener, zipper connection, screw connection, clamp connection, plug connection, snap connection and strap connection

7. A toilet device (1) according to one of the preceding claims, **characterized in that** the capacitive sensor (4) allows measurement of parameters present outside the housing (2), such as information on the localization of the user of the toilet device and temporal and spatial changes.

8. A toilet device (1) according to one of the preceding claims, **characterized in that** it additionally comprises a temperature sensor (12) and/or an acceleration sensor.

9. A toilet device (1) according to one of the preceding claims, **characterized in that** the temperature sensor (12) is set up for contactless measurement of the urine temperature and preferentially has one or more of the following properties:
• The temperature sensor (12) is set up to allow measurement of the urine temperature during urination;
• The temperature sensor (12) is set up to allow measurement of the surface temperature of the interior of the housing and its temporal change;
• The temperature sensor (12) is an infrared sensor;
• The temperature sensor (12) is a 1D, 2D or 3D sensor.

10. A process for measuring micturition parameters in a toilet device (1) comprising a housing (2) with a housing opening (10) and a capacitative proximity sensor (4), wherein the process comprises the following steps:
a) Contactless measurement of the micturition parameters during urination by a capacitative proximity sensor (4);
b) Optimal measurement of the urine temperature during urination through an optional additional temperature sensor (12) installed in the toilet device for this option;
c) Optional measurement of user movement data, vibration and structure-borne sound during urination through an additional acceleration sensor installed in the toilet device for this option;
d) Wired or wireless transmission of the data collected in steps a) to c) to an evaluation unit (6);
e) Evaluation of measurement data in the evaluation unit.

11. A process according to Claim 10, **characterized in that** the process is performed with a toilet device (1) according to one of Claims 1 to 10.

12. A process according to Claim 10 or 11, **characterized in that** evaluation of the measurement data in the evaluation unit (6) is model-based or occurs by means of calibration curves, the model-based evaluation preferentially being based on a mathematical, physical, physiological or medical model or using pattern recognition algorithms.

13. A process according to Claims 10 to 12, **characterized in that** the toilet device (1) additionally has a toilet flush whose characteristics such as flush water quantity and/or flush duration and/or flush water temperature are used as reference values for the evaluation.

14. Use of a capacitative proximity sensor (4) for measuring micturition parameters in a toilet device (1),
whereby the capacitative proximity sensor reacts contactlessly to the approach of fluids,
whereby the toilet device (1) is selected from the group comprising toilet, urinal, bidet, commode, squat toilet and chamber pot.

## Revendications

1. Dispositif de toilettes (1) destiné à mesurer des paramètres de miction, comprenant :
• un corps (2) présentant une ouverture de corps (10) destinée à recevoir de l'urine ;
• un capteur capacitif (4) destiné à mesurer des paramètres de miction en fonction du temps,
dans lequel le capteur capacitif (4) est un capteur de proximité capacitif qui réagit sans contact à l'approche de fluides,
dans lequel le dispositif de toilettes (1) est sélectionné dans le groupe composé de toilettes, d'un urinoir, d'un bidet, d'une chaise percée, de toilettes à la turque et d'un pot de chambre.

2. Dispositif de toilettes (1) selon la revendication 1, **caractérisé en ce que** les paramètres de miction mesurés sont sélectionnés dans le groupe composé du débit d'urine, du volume de miction, de la durée de miction, de la fréquence de miction, de la vitesse de miction, de la pression de vessie, de la pression de plancher pelvien, de la largeur de voie urétrale et de caractéristiques de miction telles qu'une miction intermittente ou un écoulement goutte à goutte.

3. Dispositif de toilettes (1) selon l'une des revendications 1 à 2, **caractérisé en ce que** le capteur capacitif (4) du dispositif pour toilettes (1) est posé dans une ou plusieurs des positions suivantes :
• au niveau de la paroi interne du corps,
• au niveau de la paroi externe du corps,
• à l'intérieur de la paroi du corps,
• au niveau de l'ouverture de corps (10),
l'application au niveau de la paroi externe du corps étant préférée.

4. Dispositif de toilettes (1) selon l'une des revendications précédentes, **caractérisé en ce que** le capteur capacitif (4) présente une ou plusieurs des propriétés suivantes :
• une épaisseur de capteur comprise entre 1 nm et 20 mm,
• le capteur comprend une couche de support inférieure, une couche centrale comportant l'électrode et la connexion, et une couche de montage supérieure permettant de relier le capteur au corps ou à l'ouverture de corps, la couche de support et la couche de montage se composant de préférence d'un matériau électriquement isolant ;
• le capteur est inséré dans une matrice de support isolante, telle qu'un plastique ou un gel solide, la surface de la matrice de support présentant au moins partiellement une couche de colle ;
• le capteur comprend un moyen d'assemblage permettant une connexion électronique ou électrique sélectionnée de préférence dans le groupe présentant un boutonpoussoir métallique, un assemblage adhésif, un câble et une fiche d'un connecteur ;
• le capteur comprend un module de transmission de données permettant la transmission de données filaire ou sans fil ;
• le capteur est conçu sous forme de film ;
• le capteur est directement déposé en phase vapeur sur le corps ou gravé dans le matériau de corps ;
• le capteur comprend des électrodes formées d'un mince film métallique, qui sont appliquées sur une couche électriquement isolante composée par exemple de caoutchouc ou d'un matériau plastique flexible tel qu'un film de polyimide.

5. Dispositif de toilettes (1) selon l'une des revendications précédentes, **caractérisé en ce que** plusieurs capteurs capacitifs (4) sont posés dans le dispositif de toilettes (1) de façon telle qu'ils permettent une mesure bidimensionnelle ou tridimensionnelle de la miction, et sont à cet effet disposés de préférence dans ou contre le corps en tant que matrice 2D ou matrice 3D.

6. Dispositif de toilettes (1) selon l'une des revendications précédentes, **caractérisé en ce que** le capteur capacitif (4) est relié au corps (2) de façon fixe, amovible sous condition ou amovible de façon réversible, une liaison amovible de façon réversible étant préférée, et la liaison amovible de façon réversible étant de façon particulièrement préférée sélectionnée dans le groupe présentant : une ventouse, un assemblage collé, un assemblage par adhésion sans colle, un assemblage magnétique, une fermeture velcro, une fermeture à glissière, un assemblage vissé, un assemblage par serrage, un raccord mâle et femelle, un assemblage par encliquetage et un assemblage par courroie.

7. Dispositif de toilettes (1) selon l'une des revendications précédentes, **caractérisé en ce que** le capteur capacitif (4) permet la mesure de paramètres existant à l'extérieur du corps (2), comme par exemple des informations relatives à la localisation de l'utilisateur du dispositif de toilettes et son évolution temporelle et spatiale.

8. Dispositif de toilettes (1) selon l'une des revendications précédentes, **caractérisé en ce qu'**il comprend en outre un capteur de température (12) et/ou un capteur d'accélération.

9. Dispositif de toilettes (1) selon l'une des revendications précédentes, **caractérisé en ce que** le capteur de température (12) est conçu pour mesurer sans contact la température de l'urine, et présente de préférence une ou plusieurs des propriétés suivantes :
• le capteur de température (12) est conçu de façon telle qu'il permet la mesure de la température de l'urine pendant la miction ;
• le capteur de température (12) est conçu de façon telle qu'il permet la mesure de la température de surface du côté intérieur du corps de toilettes et de son évolution temporelle ;
• le capteur de température (12) est un capteur infrarouge ;
• le capteur de température (12) est un capteur 1D, 2D ou 3D.

10. Procédé de mesure de paramètres de miction dans un dispositif de toilettes (1) comportant un corps (2) comportant une ouverture de corps (10), et un capteur de proximité capacitif (4), le procédé comprenant les étapes suivantes :
a) mesure sans contact des paramètres de miction pendant la miction par le capteur de proximité capacitif (4) ;
b) mesure optionnelle de la température de l'urine pendant la miction via un capteur de température (12) éventuellement posé en plus pour cette option dans le dispositif de toilettes ;
c) mesure optionnelle des données de mouvement, des vibrations et du bruit corporel côté utilisateur pendant la miction via un capteur d'accélération éventuellement posé en plus pour cette option dans le dispositif de toilettes ;
d) transmission filaire ou sans fil des données de mesure obtenues dans les étapes a) à c) à une unité d'évaluation (6) ;
e) évaluation des données de mesure dans l'unité d'évaluation.

11. Procédé selon la revendication 10, **caractérisé en ce que** le procédé est réalisé avec un dispositif de toilettes (1) selon l'une des revendications 1 à 10.

12. Procédé selon la revendication 10 ou 11, **caractérisé en ce que** l'évaluation des données de mesure est réalisée dans l'unité d'évaluation (6) sur la base d'un modèle ou via des courbes d'étalonnage, l'évaluation sur la base d'un modèle reposant de préférence sur un modèle mathématique, physique, physiologique ou médical ou se déroulant par l'intermédiaire d'algorithmes de reconnaissance de formes.

13. Procédé selon les revendications 10 à 12, **caractérisé en ce que** le dispositif de toilettes (1) présente de plus une chasse-d'eau pour WC dont les caractéristiques telles que la quantité d'eau de chasse et/ou la durée de chasse et/ou la température de l'eau de chasse servent de valeurs de référence pour l'évaluation.

14. Utilisation d'un capteur de proximité capacitif (4) pour mesurer des paramètres de miction dans un dispositif de toilettes (1),
le capteur de proximité capacitif réagissant sans contact à l'approche de fluides, dans lequel le dispositif de toilettes (1) est sélectionné dans le groupe composé de toilettes, d'un urinoir, d'un bidet, d'une chaise percée, de toilettes à la turque et d'un pot de chambre.
